# EUROPEAN PATENT APPLICATION

(11) **EP 4 079 219 A1**
(43) Date of publication of application: **26.10.2022**
(21) Application number: 20901918.1
(22) Date of filing: 13.11.2020
(51) Int. Cl.: A61B 5/02, A61B 5/16

(54) **BIOLOGICAL INFORMATION MEASUREMENT APPARATUS AND BIOLOGICAL INFORMATION MEASUREMENT METHOD**

(30) Priority: 19.12.2019 JP 2019229494
(71) Applicant: Sony Group Corporation, Tokyo 108-0075 (JP)
(72) Inventor: IKUTA Tomoya, Tokyo 108-0075 (JP); ITO Atsushi, Tokyo 108-0075 (JP); SAZUKA Naoya, Tokyo 108-0075 (JP)
(74) Representative: D Young & Co LLP
(86) International application number: PCT/JP2020/042385
(87) International publication number: WO 2021/124749

(57) **Abstract**

The present technology provides a living body information measurement apparatus and a living body information measuring method that make it possible to discriminate a state of a living body with high accuracy.

The present technology provides a living body information measurement apparatus including: a sensor device that applies light to a living body and individually detects light scattered by a plurality of parts in the living body; and a processor that discriminates a state of the living body on the basis of outputs of the sensor device for the respective parts. According to the present technology, it is possible to provide a living body information measurement apparatus and a living body information measuring method that make it possible to discriminate the state of the living body with high accuracy.

## Description

### Technical Field

The technology according to the present disclosure (hereinafter also referred to as "the present technology") relates to a living body information measurement apparatus and a living body information measuring method.

### Background Art

There has been known a technology of measuring living body information such as a pulse wave by applying light to a living body and receiving the light having passed through the inside of the living body.

For example, PTL 1 discloses a pulse wave sensor that measures a plurality of plethysmogram signals and selects a signal having highest intensity from among them, or an average value or a total value of a plurality of signals.

For example, PTL 2 discloses an intrathoracic pressure estimation device that measures a plurality of plethysmogram signals from the same part in a living body and estimates an intrathoracic pressure on the basis of a difference between intrathoracic pressures corresponding to the plurality of plethysmogram signals.

### Citation List

### Patent Literature

PTL 1: Japanese Unexamined Patent Application Publication No. 2012-19929
PTL 2: Japanese Unexamined Patent Application Publication No. 2018-68556

### Summary of the Invention

### Problems to be Solved by the Invention

However, even if existing measuring techniques are used, it is not possible to discriminate a state of a living body with high accuracy.

Accordingly, it is a main object of the present technology to provide a living body information measurement apparatus and a living body information measuring method that make it possible to discriminate the state of the living body with high accuracy.

### Means for Solving the Problems

The present technology provides a living body information measurement apparatus including:
a sensor device that applies light to a living body and individually detects light scattered by a plurality of parts in the living body; and
a processor that discriminates a state of the living body on the basis of outputs of the sensor device for the respective parts.

The living body information measurement apparatus according to claim 1, in which the sensor device includes at least one light-emitting element and a plurality of light-receiving elements, or a plurality of light-emitting elements and at least one light-receiving element.

The processor may include an extracting section that extracts information about the plurality of parts from the outputs of the sensor device, and may discriminate the state of the living body on the basis of the information about the plurality of parts extracted by the extracting section.

The processor may discriminate the state of the living body with use of information about the respective parts extracted in time series by the extracting section.

The processor may include a recording section that records the information about the plurality of parts extracted by the extracting section.

The processor may include a machine learning section that performs machine learning of the information about the plurality of parts extracted by the extracting section.

The processor may include an analyzer that analyzes the information about the plurality of parts extracted by the extracting section and performs determination of the state of the living body on the basis of a result of such analysis.

The analyzer may acquire an interrelation of the information about the plurality of parts extracted by the extracting section, and may perform the discrimination on the basis of a thus-acquired result.

The analyzer may calculate a difference between information about each of the parts extracted by the extracting section and information about that part extracted by the extracting section before extracting that information, and may perform discrimination of the state of the living body on the basis of a result of such calculation.

The analyzer may acquire an interrelation between the differences in the information about the plurality of parts, and may perform discrimination of the state of the living body on the basis of a thus-acquired result.

The analyzer may acquire a plurality of the interrelations, and may perform determination of the state of the living body on the basis of a thus-acquired result.

The analyzer may acquire an interrelation between the differences in the information about the plurality of parts, and may perform discrimination of the state of the living body on the basis of a thus-acquired result and the differences for the respective parts.

The processor may include a determining section that determines whether or not the information about the plurality of parts extracted by the extracting section satisfies a condition indicating that the state of the living body is a predetermined state, and the recording section may hold or update the information for the respective parts when a result of determination in the determining section is affirmative.

The information about the plurality of parts recorded on the recording section may be recorded in association with a predetermined state of the living body by performing calibration in advance with use of the living body information measurement apparatus.

The extracting section may include a lowpass filter.

The extracting section may include an envelope detector.

A plurality of the light-emitting elements may be included, the plurality of the light-emitting elements may apply light to the living body at different timings, and the processor may acquire, from a plurality of signals outputted by the light-receiving element at different timings, information about the plurality of parts corresponding the signals.

A plurality of the light-receiving elements may be included, and the processor may acquire, from signals outputted from the plurality of the light-receiving elements, information about the plurality of parts corresponding to the signals.

The light-emitting elements and the light-receiving elements may be disposed in a same housing.

The plurality of parts may include a plurality of blood vessels different from each other.

The state of the living body may include a state of autonomic nerves of the living body.

The present technology also provides a living body information measuring method including:
a step of applying light to a living body and individually detecting light scattered by a plurality of parts in the living body; and
a step of discriminating a state of the living body on the basis of a detection result for each of the parts in the detecting step.

### Brief Description of Drawings

[FIG. 1] FIG. 1A is a plan view of a configuration example of a sensor device of a living body information measurement apparatus according to a first embodiment of the present technology. FIG. 1B is a cross-sectional view of a configuration example of the living body information measurement apparatus according to the first embodiment of the present technology.
[FIG. 2] FIG. 2 is a block diagram illustrating a schematic configuration of a processor of the living body information measurement apparatus according to the first embodiment of the present technology.
[FIG. 3] FIG. 3 is a block diagram illustrating a configuration of a processor of a living body information measurement apparatus according to Example 1 of the first embodiment of the present technology.
[FIG. 4] FIG. 4 is a flowchart for describing an operation of the living body information measurement apparatus according to Example 1 of the first embodiment of the present technology.
[FIG. 5] FIG. 5 is a block diagram illustrating a configuration of a processor of a living body information measurement apparatus according to Example 2 of the first embodiment of the present technology.
[FIG. 6] FIG. 6 is a flowchart for describing an operation of the living body information measurement apparatus according to Example 2 of the first embodiment of the present technology.
[FIG. 7] FIG. 7 is a block diagram illustrating a configuration of a processor of a living body information measurement apparatus according to Example 3 of the first embodiment of the present technology.
[FIG. 8] FIG. 8 is a flowchart for describing an operation of the living body information measurement apparatus according to Example 3 of the first embodiment of the present technology.
[FIG. 9] FIG. 9 is a block diagram illustrating a configuration of a processor of a living body information measurement apparatus according to Example 4 of the first embodiment of the present technology.
[FIG. 10] FIG. 10 is a flowchart for describing an operation of the living body information measurement apparatus according to Example 4 of the first embodiment of the present technology.
[FIG. 11] FIG. 11 is a block diagram illustrating a configuration of a processor of a living body information measurement apparatus according to Example 5 of the first embodiment of the present technology.
[FIG. 12] FIG. 12 is a block diagram illustrating a configuration of a processor of a living body information measurement apparatus according to Example 6 of the first embodiment of the present technology.
[FIG. 13] FIG. 13A is a plan view of a configuration example of a sensor device of a living body information measurement apparatus according to a second embodiment of the present technology. FIG. 13B is a cross-sectional view of a configuration example of the sensor device of the living body information measurement apparatus according to the second embodiment of the present technology.
[FIG. 14] FIG. 14 is a plan view of a configuration of a sensor device of a living body information measurement apparatus according to Example 1 of a third embodiment of the present technology.
[FIG. 15] FIG. 15 is a plan view of a configuration of a sensor device of a living body information measurement apparatus according to Example 2 of the third embodiment of the present technology.
[FIG. 16] FIG. 16 is a plan view of a configuration of a sensor device of a living body information measurement apparatus according to Example 3 of the third embodiment of the present technology.

### Modes for Carrying Out the Invention

The following describes preferred embodiments of the present technology in detail with reference to the accompanying drawings. It is to be noted that in the present specification and drawings, components having substantially the same functional configurations are denoted by the same reference numerals, and repetitive description thereof is omitted. The embodiments described below are illustrative of representative embodiments of the present technology and are not to be construed as limiting the scope of the present technology. In this specification, each of a living body information measurement apparatus and a living body information measuring method according to the present technology may have at least one effect even in a case where it is described that each of the living body information measurement apparatus and the living body information measuring method according to the present technology has a plurality of effects. The effects described herein are merely illustrative and non-limiting, and may have other effects.

Further, description is given in the following order.
1. Introduction
2. Living Body Information Measurement Apparatus According to First Embodiment of Present Technology
3. Living Body Information Measurement Apparatus According to Second Embodiment of Present Technology
4. Living Body Information Measurement Apparatus According to Third Embodiment of Present Technology
5. Modification Examples of Present Technology

### 1. <Introduction>

Currently, there are a large number of measurement apparatuses that acquire living body information from a living body (e.g., a human body) in the world. Among them, apparatuses that measure a pulse as pulse sensors have been in widespread use as watch type and wrist-band type devices with evolution of wearable devices, and a photoplethysmography (photoplethysmography; PPG) system has been widely adopted.

In addition, apparatuses that measure blood flow velocity have been in widespread use in the medical field and the like, and a laser doppler flowmeter (Laser Doppler Flowmetry; LDF) and the like are known. Living body information such as a plethysmogram and blood flow velocity reflects change in a cardiovascular system of the living body, and is therefore extremely effective living body information for observation of the state of the living body (e.g., a state of autonomic nerves).

### 2. <Living Body Information Measurement Apparatus According to First Embodiment of Present Technology>

Description is given of a living body information measurement apparatus according to a first embodiment of the present technology with reference to drawings.

The living body information measurement apparatus is an apparatus that is attached to, for example, skin of a living body (e.g., a human body) to discriminate a state of the living body (e.g., a state of autonomic nerves of the living body, or the like).

As a form of the living body information measurement apparatus, for example, a variety of forms such as a wrist-band type, an earring type, a ring type, a necklace type, a stick-on type, a supporter type are assumed.

Description is given below of the living body information measurement apparatus according to the first embodiment of the present technology (living body information measurement apparatuses according to Examples 1 to 5).

### [Living Body Information Measurement Apparatus According to Example 1]

### <<Configuration of Living Body Information Measurement Apparatus According to Example 1>>

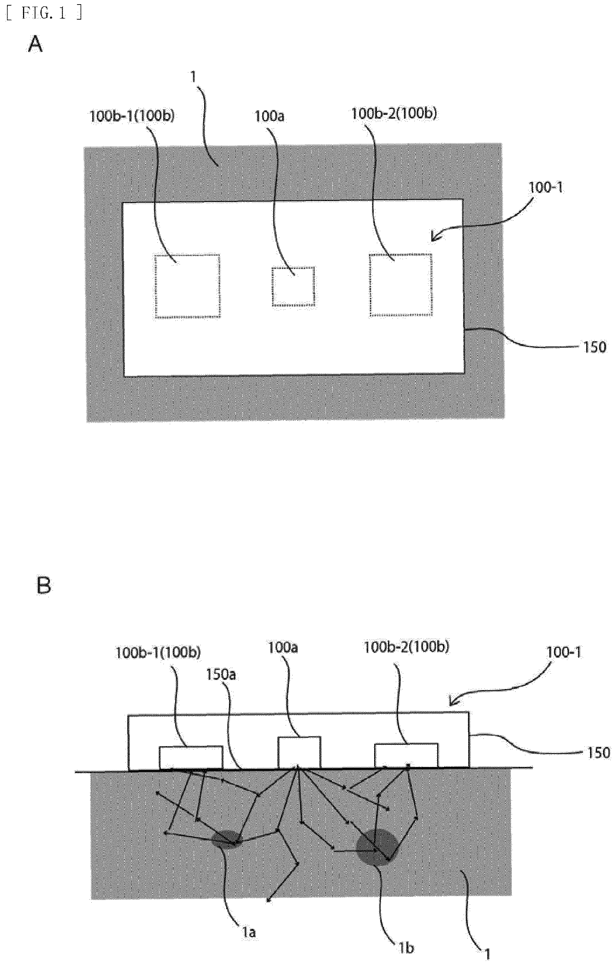

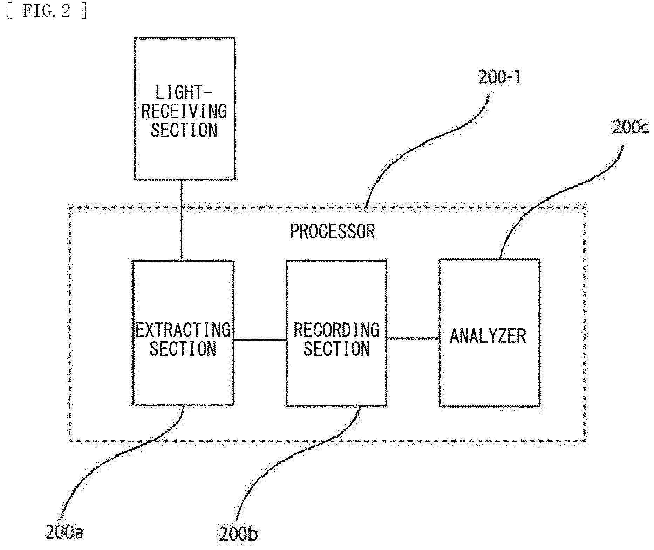

FIG. 1A is a plan view of a configuration of a sensor device of the living body information measurement apparatus According to Example 1. FIG. 1B is a cross-sectional view of a configuration of the living body information measurement apparatus according to Example 1 of the first embodiment of the present technology. FIGs. 1A and 1B illustrate a state in which the sensor device is attached to skin of a living body. FIG. 2 is a block diagram illustrating a schematic configuration of a processor of the living body information measurement apparatus according to Example 1 of the first embodiment of the present technology.

The living body information measurement apparatus according to Example 1 includes a sensor device 100-1 and a processor 200-1 as illustrated in FIGs. 1 and 2.

### <Sensor Device>

The sensor device 100-1 applies light to a living body 1 (e.g., a human body), and individually detects light scattered by a plurality of (e.g., two) parts (e.g., blood vessels) in the living body 1.

For example, the sensor device 100-1 is able to apply light to the living body 1 and separately detect light scattered by one part (e.g., a blood vessel 1a, see FIG. 1B) in the living body 1 and light scattered by another part (e.g., a blood vessel 1b, see FIG. 1B).

It is to be noted that examples of the living body 1 include bodies of animals and the like other than a human, in addition to the human body.

The sensor device 100-1 includes a light source section and a light-receiving section.

The light source section includes at least one (e.g., one) light-emitting element 100a.

The light-receiving section includes a plurality of (e.g., two) light-receiving elements 100b (e.g., first and second light-receiving elements 100b-1 and 100b-2).

As illustrated in FIG. 1A and FIG. 1B, the light-emitting element 100a and each of the light-receiving elements 100b are disposed in the same housing 150. The housing 150 has a light transmission window 150a in a bottom wall section that is a wall section on side to be attached to the living body 1. The light transmission window 150a allows at least a portion of light from the light-emitting element 100a to pass therethrough.

The light-emitting element 100a and the two light-receiving elements 100b are provided on the light transmission window 150a in the housing 150.

The two light-receiving elements 100b are disposed around the light-emitting element 100a.

More specifically, the two light-receiving elements 100b are disposed, for example, at positions between which the light-emitting element 100a is sandwiched.

Examples of the light-emitting element 100a include laser light sources such as a LED (light-emitting diode), an LD (edge emitting laser), and a VCSEL (surface emitting laser).

For example, in a case where the light-emitting element 100a is a laser light source that emits coherent light, it is possible to use the light-emitting element 100a for measurement of living body information such as a plethysmogram and blood flow velocity.

For example, in a case where the light-emitting element 100a is an LED that emits incoherent light, it is possible to use the light-emitting element 100a for measurement of living body information such as a plethysmogram.

Light emitted from the light-emitting element 100a may be visible light or invisible light (e.g., infrared light).

The light-receiving element 100b includes, for example, a PD (photodiode), a phototransistor, and the like.

Specifically, the light-receiving element 100b may be a divided PD having a plurality of light reception regions that are one-dimensionally or two-dimensionally arranged, a line sensor in which, for example, pixels including PDs are one-dimensionally arranged, or an image sensor in which, for example, pixels including PDs are two-dimensionally arranged.

For example, as illustrated in FIG. 1B, the sensor device 100-1 configured as described above is attached to the living body 1 to make a direction of arrangement of the light-emitting element 100a and the plurality of light-receiving elements 100b substantially coincident with a direction of arrangement of two blood vessels 1a and 1b in the living body 1 and to position the light-emitting element 100a between the two blood vessels 1a and 1b as viewed from a direction facing the living body 1.

In a case where the light-emitting element 100a emits light in this state, the light passes through the light transmission window 150a to be applied to the living body 1.

A portion of light that has been applied to the living body 1 and has entered the living body 1 propagates in the living body 1 to enter the blood vessel 1a, and is scattered by the blood vessel 1a. At least a portion of the light scattered by the blood vessel 1a propagates in the living body 1 to enter the first light-receiving element 100b-1.

In contrast, another portion of the light that has been applied to the living body 1 and has entered the living body 1 propagates in the living body 1 to enter the blood vessel 1b, and is scattered by the blood vessel 1b. A portion of the light scattered by the blood vessel 1b propagates in the living body 1 to enter the second light-receiving element 100b-2.

Thus, the sensor device 100-1 is able to individually detect the light scattered by each of the two blood vessels 1a and 1b in the living body 1.

That is, the sensor device 100-1 is able to separately detect the light scattered by the blood vessel 1a and the light scattered by the blood vessel 1b.

In this case, a signal outputted from the first light-receiving element 100b-1 has a waveform corresponding to pulsation of the blood vessel 1a, and a signal outputted from the second light-receiving element 100b-2 has a waveform corresponding to pulsation of the blood vessel 1b. Pulsation of a blood vessel is an index for determining a state (e.g., a state of autonomic nerves) of the living body 1.

### <Processor>

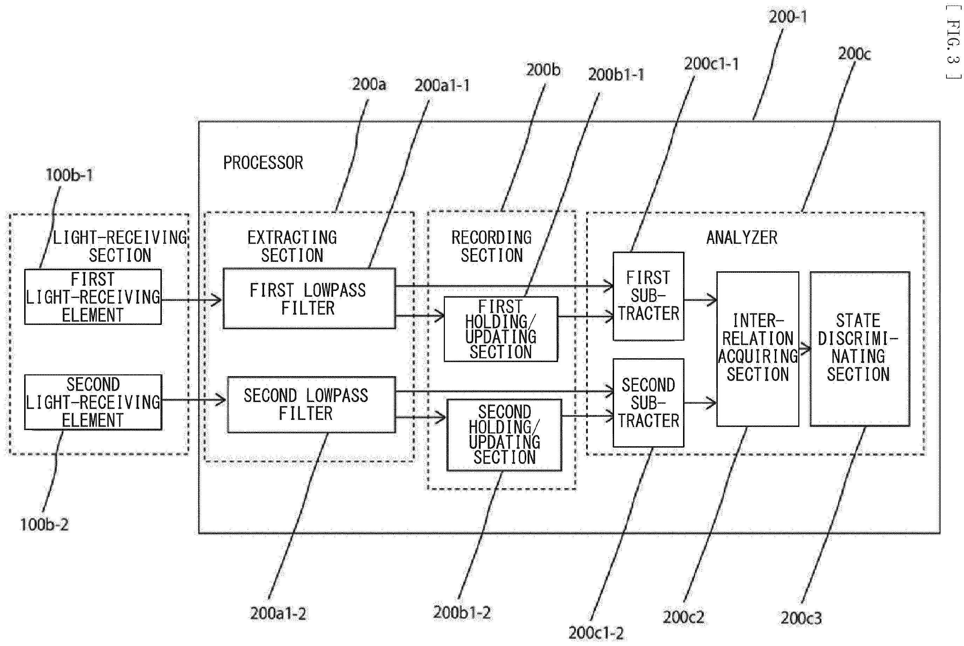

FIG. 3 is a block diagram illustrating a function of the processor 200-1.

The processor 200-1 discriminates the state (e.g., the state of autonomic nerves) of the living body 1 on the basis of outputs of the sensor device 100-1 for respective parts (e.g., blood vessels) of the living body 1.

The processor 200-1 includes an extracting section 200a, a recording section 200b, and an analyzer 200c. The processor 200-1 further includes a controller that integrally controls respective components.

The processor 200-1 may be provided in the housing 150 and be coupled to the sensor device 100-1, may be provided outside the housing 150 integrally with the housing 150 and be coupled to the sensor device 100-1, or may be provided outside the housing 150 and be wiredly or wirelessly coupled to the sensor device 100-1.

### (Extracting Section)

The extracting section 200a extracts information about a plurality of parts (e.g., the blood vessels 1a and 1b) from outputs of the sensor device 100-1. As illustrated in FIG. 3, the extracting section 200a is implemented by a plurality of (e.g., two) lowpass filters (e., first and second lowpass filters 200a1-1 and 200a1-2).

The first lowpass filter 200a1-1 is coupled to an output end of the first light-receiving element 100b-1, and cuts a high-frequency component (a noise component) of a signal outputted from the first light-receiving element 100b-1 to output the signal from which the high-frequency component is cut.

The signal having passed through first lowpass filter 200a1-1 is transmitted to the recording section 200b and the analyzer 200c.

The second lowpass filter 200a1-2 is coupled to an output end of the second light-receiving element 100b-2, and cuts a high-frequency component (a noise component) of a signal outputted from the second light-receiving element 100b-2 to output the signal from which the high-frequency component is cut.

The signal having passed through the second lowpass filter 200a1-2 is transmitted to the recording section 200b and the analyzer 200c.

### (Recording Section)

The recording section 200b records information about the plurality of parts (e.g., the blood vessels 1a and 1b) extracted by the extracting section 200a. The recording section 200b is implemented by, for example, a CPU, a memory such as a ROM, a RAM, or a flash memory, a hard disk, and the like.

The recording section 200b includes, for example, a first holding/updating section 200b1-1 and a second holding/updating section 200b1-2.

The first holding/updating section 200b1-1 is coupled to an output end of the first lowpass filter 200a1-1, and holds or updates the signal having passed through the first lowpass filter 200a1-1.

The second holding/updating section 200b1-2 is coupled to an output end of the second lowpass filter 200a1-2, and holds or updates the signal having passed through the second lowpass filter 200a1-2.

The controller described above predetermines the state of the living body 1 (e.g., the state of autonomic nerves of the living body 1) on the basis of, for example, the signal having passed through the first lowpass filter 200a1-1.

Specifically, for example, the controller described above calculates a heart rate from the signal having passed through the first lowpass filter 200a1-1 or the signal having passed through the second lowpass filter 200a1-2, and in a case where a result of such calculation is less than a threshold, the controller determines that the state of autonomic nerves of the living body 1 is a rest state.

The controller described above causes the first holding/updating section 200b1-1 to hold or update only a signal from which it is determined that the state of autonomic nerves of the living body 1 is the rest state, among signals having passed through the first lowpass filter 200a1-1. In this case, the signal held or updated by the first holding/updating section 200b1-1 satisfies a condition of the rest state.

The controller described above causes the second holding/updating section 200b1-2 to hold or update only a signal from which it is determined that the state of autonomic nerves of the living body 1 is the rest state, among signals having passed through the first lowpass filter 200a1-2. In this case, the signal held or updated by the second holding/updating section 200b1-2 satisfies the condition of the rest state.

It is to be noted that, for example, the controller described above may calculate a heart rate from the signal having passed through the first lowpass filter 200a1-1 or the signal having passed through the second lowpass filter 200a1-2, and in a case where a result of such calculation is equal to or greater than the threshold described above, the controller may determine that the state of autonomic nerves of the living body 1 is a tension state.

In a case where such determination is made, for example, the controller described above may hold or update only a signal from which it is determined that the state of autonomic nerves of the living body 1 is the tension state, among signals having passed through the first lowpass filter 200a1-1. In this case, the signal held or updated by the first holding/updating section 200b1-1 satisfies a condition of the tension state.

In a case where such determination is made, for example, the controller described above may hold or update only a signal from which it is determined that the state of autonomic nerves of the living body 1 is the tension state, among signals having passed through the second lowpass filter 200a1-2. In this case, the signal held or updated by the second holding/updating section 200b1-2 satisfies the condition of the tension state.

Here, a signal of each channel (a path of a signal outputted from each light-receiving element) has a wavelength corresponding to a motion of a different part (e.g., a blood vessel) of the living body 1, and it is conceivable that a heart rate calculated from that signal has an approximate value.

Accordingly, for example, the controller described above may calculate a heart rate from the signal having passed through the first lowpass filter 200a1-1, and may calculate a heart rate from the signal having passed through the second lowpass filter 200a1-2, and the controller may determine that the state of autonomic nerves of the living body 1 is the rest state in a case where an average value of both results of calculation is less than a threshold, and the state of autonomic nerves of the living body 1 is the tension state in a case where the average value is equal to or greater than the threshold. This makes it possible to calculate the heart rate with high accuracy and improve a system of determining the state of autonomic nerves.

### (Analyzer)

The analyzer 200c analyzes at least information about a plurality of parts (e.g., the blood vessels 1a and 1b) recorded on the recording section 200b, and discriminates the state of the living body 1 (e.g., the state of autonomic nerves of the living body 1) on the basis of a result of such analysis. The analyzer 200c is implemented together with the controller described above by a CPU (Central Processing Unit), a FPGA (Field Programmable Gate Array), and the like.

To be more specific, the analyzer 200c acquires a difference between information about each part (e.g., each blood vessel) recorded on the recording section 200b and information about that part (e.g., that blood vessel) extracted by the extracting section 200a after recording that information on the recording section 200b, and performs discrimination of the state of the living body 1 on the basis of a thus-acquired result.

To be more specific, the analyzer 200c acquires an interrelation between differences in information for respective parts of the living body 1, and discriminates the state of the living body 1 on the basis of a thus-acquired result.

Examples of the "interrelation" described above include a characteristic amount such as dispersion, standard deviation, a difference, skewness, kurtosis, correlation, a quantile, an average value, and a median value.

The analyzer 200c includes a first subtracter 200c1-1, a second subtracter 200c1-2, an interrelation acquiring section 200c2, and a state discriminating section 200c3.

The first subtracter 200c1-1 is coupled to the output end of the first lowpass filter 200a1-1 and the first holding/updating section 200b1-1. The first subtracter 200c1-1 subtracts a signal (an old signal) recorded (held or updated) on the first holding/updating section 200b1-1 from a signal (a new signal) transmitted through the first lowpass filter 200a1-1 after a lapse of a predetermined time from the time of such recording, and outputs a result of such subtraction (a difference).

The second subtracter 200c1-2 is coupled to the output end of the second lowpass filter 200a1-2 and the second holding/updating section 200b1-2.The second subtracter 200c1-2 subtracts a signal (an old signal) recorded (held or updated) on the second holding/updating section 200b1-2 from a signal (a new signal) transmitted through the second lowpass filter 200a1-2 after a lapse of a predetermined time from the time of such recording, and outputs a result of such subtraction (a difference).

The interrelation acquiring section 200c2 is coupled to an output end of the first subtracter 200c1-1 and an output end of the second subtracter 200c1-2.

The interrelation acquiring section 200c2 acquires an interrelation between the result of subtraction (the difference) in the first subtracter 200c1-1 and the result of subtraction (the difference) in the second subtracter 200c1-2 (calculates the characteristic amount described above), and outputs a thus-acquired result (a result of calculation).

The state discriminating section 200c3 is coupled to an output end of the interrelation acquiring section 200c2.

The state discriminating section 200c3 discriminates the state of the living body 1 on the basis of the interrelation acquired by the interrelation acquiring section 200c2.

Here, a signal of each channel has a waveform corresponding to pulsation (a behavior) of a corresponding blood vessel. Pulsation of blood vessels differs for each blood vessel; therefore, the waveform of a signal acquired for each blood vessel also differs. However, the waveform of the result of subtraction (the waveform of the difference) involving new and old signals of each channel described above is information that reflects presence or absence of change in the state of autonomic nerves independent of a signal waveform for each blood vessel.

It is conceivable that while the state of autonomic nerves continues in the same state, waveforms of differences between signals of respective channels are waveforms approximate to each other. Accordingly, in a case where the waveforms of differences between the signals of the respective channels are approximate to each other, it is presumable that the state of autonomic nerves continues in the same state.

Accordingly, in a case where the acquired result (interrelation) in the interrelation acquiring section 200c2, that is, the characteristic amount described above is, for example, less than a threshold, the state discriminating section 200c3 determines that the state of autonomic nerves continues in the same state, and outputs a result of discrimination (e.g., the rest state or the tension state) of the state of autonomic nerves corresponding to such result of determination.

In contrast, it is conceivable that in a case where the state of autonomic nerves changes, the waveforms of the differences between the new and old signals of the respective channels are waveforms not approximate to each other. Accordingly, in a case where the waveforms of differences between the signals of the respective channels are not approximate to each other, it is presumable that the state of autonomic nerves has changed.

Accordingly, in a case where the acquired result (interrelation) in the interrelation acquiring section 200c2, that is, the characteristic amount described above is, for example, equal to or greater than the threshold, the state discriminating section 200c3 determines that the state of autonomic nerves has changed, and outputs a result of discrimination (e.g., the rest state or the tension state) of the state of autonomic nerves corresponding to such result of determination.

### <<Operation of Living Body Information Measurement Apparatus According to Example 1>>

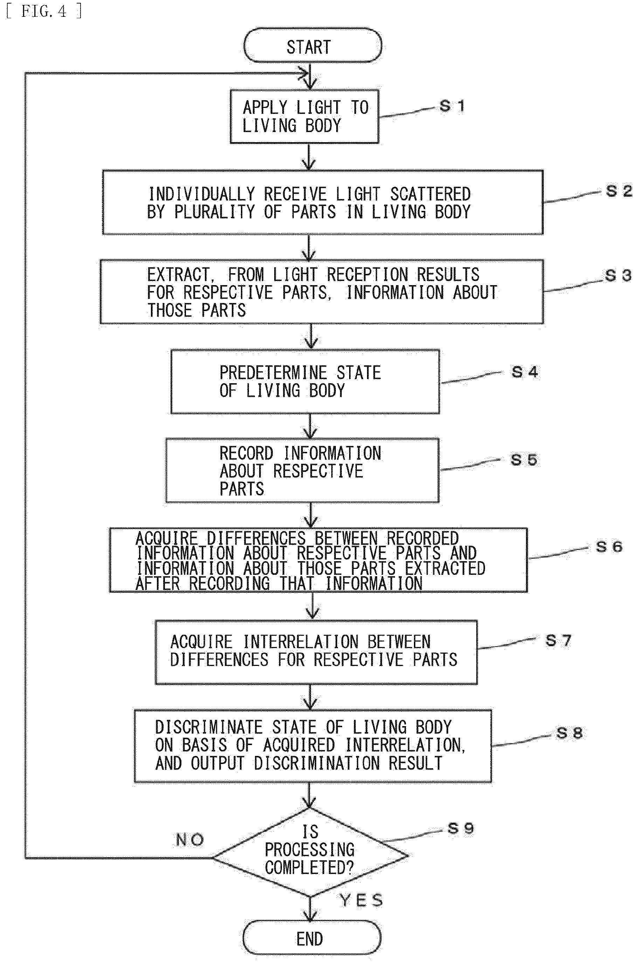

Description is given below of an operation of the living body information measurement apparatus according to Example 1 (an example of the living body information measuring method according to the present technology) with reference to a block diagram in FIG. 3 and a flowchart in FIG. 4.

It is assumed that the sensor device 100-1 of the living body information measurement apparatus according to Example 1 is attached to the living body 1 as illustrated in FIG. 1B, for example.

In a first step S1, the light-emitting element 100a applies light to the living body 1. Specifically, when the living body information measurement apparatus is activated, the light-emitting element 100a emits light to apply the light to the living body 1.

In the next step S2, a plurality of light-receiving elements 100b individually receives light scattered by a plurality of parts (a plurality of blood vessels 1a and 1b) in the living body 1.

In the next step S3, the extracting section 200a extracts, from output signals of the light-receiving elements 100b, information about respective corresponding parts.

Specifically, an output signal of the first light-receiving element 100b-1 is filtered by the first lowpass filter 200a1-1 to cut a high-frequency component (a noise component), thereby extracting pulsation (a pulse wave) of the blood vessel 1a.

An output signal of the second light-receiving element 100b-2 is filtered by the second lowpass filter 200a1-2 to cut a high frequency, thereby extracting pulsation (a pulse wave) of the blood vessel 1b.

In the next step S4, the controller predetermines the state of the living body 1. Specifically, the controller calculates the heart rate of the living body 1 on the basis of at least one of the signal having passed through the first lowpass filter 200a1-1 or the signal having passed through the second lowpass filter 200a1-2, and predetermines the state of autonomic nerves of the living body 1 (e.g., the rest state or the tension state) in accordance with a result of such calculation.

In the next step S5, the recording section 200b records the information about the respective parts extracted by the extracting section 200a in accordance with a result of determination in the step S4.

Specifically, in a case where a result of determination that the state of autonomic nerves of the living body 1 is, for example, the rest state (or the tension state) is acquired in the step S4, the first holding/updating section 200b1-1 and the second holding/updating section 200b2 associate the information about the respective parts (e.g., pulse waves of the blood vessels 1a and 1b) extracted by the extracting section 200a with the rest state (or the tension state), and hold or update the information.

In the next step S6, the analyzer 200c subtracts the information about the respective parts recorded on the recording section 200b from information about those parts extracted by the extracting section 200a after recording that information. Specifically, the first subtracter 200c1-1 subtracts a signal recorded (held or updated) on the first holding/updating section 200b1-1 from a signal transmitted through the first lowpass filter 200a1-1 after a lapse of a predetermined time from when that signal is recorded. The second subtracter 200c1-2 subtracts a signal recorded (held or updated) on the second holding/updating section 200b 1-2 from a signal transmitted through the second lowpass filter 200a1-2 after a lapse of a predetermined time from when that signal is recorded.

In the next step S7, the analyzer 200c acquires an interrelation between results of subtraction (differences) for the respective parts. Specifically, the interrelation acquiring section 200c2 acquires an interrelation between the result of subtraction in the first subtracter 200c1-1 and the result of subtraction in the second subtracter 200c1-2.

In the next step S8, the analyzer 200c discriminates the state (e.g., the state of autonomic nerves) of the living body 1 on the basis of the interrelation acquired in the step S7, and outputs a result of such discrimination (e.g., the rest state or the tension state).

In the last step S9, whether or not processing is completed is determined. Determination here is affirmed when the operation of the living body information measurement apparatus is stopped, and is negated when the operation is maintained. In a case where the determination in the step S9 is affirmed, the flow ends, and in a case where the determination in the step S9 is negated, the flow returns to the step S1.

### <<Effects of Living Body Information Measurement Apparatus and Living Body Information Measuring Method According to Example 1>>

The living body information measurement apparatus according to Example 1 described above includes the sensor device 100-1 that applies light to the living body 1 and individually detects light scattered by a plurality of parts in the living body 1, and the processor 200-1 that discriminates the state of the living body 1 on the basis of outputs of the sensor device 100-1 for the respective parts.

This makes it possible to discriminate the state of the living body 1 on the basis of the outputs of the sensor device 100-1 for the respective parts in the living body 1, which makes it possible to discriminate the state of the living body 1 with high accuracy.

Here, in a case where the state of autonomic nerves is observed from living body information such as a plethysmogram and blood flow velocity, there has been an issue that accuracy is low in a case where the state of autonomic nerves is discriminated from, for example, only one plethysmogram or blood flow velocity.

In contrast, for example, in PTL 1, signal accuracy is improved by measuring a plurality of plethysmograms and selecting a signal having highest intensity from among them or selecting an average value or a total value of a plurality of signals.

However, in this case, signal accuracy is improved, but there is room for improvement in improving accuracy in determination of the state of autonomic nerves.

In addition, for example, in PTL 2, a plurality of plethysmograms is measured with use of a plurality of different detection methods, and a difference therebetween is calculated to acquire new information.

However, as one detection method, one light source is used to measure transmission and reflection plethysmograms; therefore, it is conceivable that blood vessels as measurement targets overlap in proximity to the light source. In addition, as another detection method, measurement of plethysmograms is performed by different wavelength light sources; therefore, it is conceivable that light-receiving element becomes the same, and blood vessels as measurement targets overlap in proximity to the light-receiving element.

As described above, in measurement techniques described in PTLs 1 and 2, plethysmogram signals have low independence, and include a large number of pieces of living body information derived from the same blood vessel information, and a difference is not defined in a case where a difference between the pieces of the living body information is calculated, and concerns rise that identification accuracy declines.

In addition, it is generally known that in a case where the state of autonomic nerves is the tension state, a different behavior is exhibited for each blood vessel; however, it is conceivable that even in a case where the state of autonomic nerves is the rest state, the behavior differs for each blood vessel.

Accordingly, for example, in PTLs 1 and 2, only a difference in living body information is observed without discriminating between the rest state and the tension state, and it is not possible to know whether a difference in behavior for each blood vessel in the rest state or a difference in behavior for each blood vessel in the tension state is acquired. Accordingly, there is room for improvement in discriminating the state of autonomic nerves with high accuracy.

The sensor device 100-1 includes at least one (e.g., one) light-emitting element 100a and a plurality of (e.g., two) light-receiving elements 100b. This allows different light-receiving elements 100b to receive light applied from the light-emitting element 100a and scattered by different parts in the living body 1.

The processor 200-1 includes the extracting section 200a that extracts information about a plurality of parts from outputs of the sensor device 100-1, and discriminates the state of the living body 1 on the basis of information about the plurality of parts extracted by the extracting section 200a. This makes it possible to discriminate the state of the living body 1 with use of the information about the plurality of parts extracted from the outputs of the sensor device 100-1 (outputs of the plurality of light-receiving elements 100b), which makes it possible to improve discrimination accuracy.

The processor 200-1 discriminates the state of the living body 1 with use of information about respective parts extracted in time series by the extracting section 200a. This makes it possible to discriminate the state of the living body 1 by capturing change in the state of the living body 1, which makes it possible to further improve the discrimination accuracy.

The processor 200-1 includes the recording section 200b that records the information about a plurality of parts extracted by the extracting section 200a. This makes it possible to hold or update the information about the plurality of parts.

The processor 200-1 includes the analyzer 200c that analyzes the information about the plurality of parts extracted by the extracting section 200a, and discriminates the state of the living body 1 on the basis of a result of such analysis. This makes it possible to further improve accuracy in discrimination of the state of the living body 1.

The analyzer 200c calculates a difference between the information about each part recorded on the recording section 200b, and information about that part extracted by the extracting section 200a after recording that information on the recording section 200b, and discriminates the state of the living body 1 on the basis of a result of such calculation. This makes it possible to acquire information that reflects change in the state of the living body 1 for each part.

The analyzer 200c acquires an interrelation between results of subtraction for information about a plurality of parts, and discriminates the state of the living body 1 on the basis of a thus-acquired result. This makes it possible to further improve accuracy in discrimination of the state of the living body 1.

The processor 200-1 includes the controller (a determining section) that determines whether or not the information about a plurality of parts extracted by the extracting section 200a satisfies a condition indicating that the state of the living body 1 is a predetermined state (e.g., the rest state or the tension state), and the recording section 200b holds or updates information about each part when a result of determination in the controller is affirmative. Accordingly, discrimination of the state of the living body 1 is performed on the basis of only the information about each part when the living body 1 is in the predetermined state (not including information about each part when the living body is in a state other than the predetermined state), which makes it possible to improve reliability in the discrimination.

The extracting section 200a includes a lowpass filter. Accordingly, the extracting section 200a extracts a signal from which a high-frequency component (a noise component) is cut from a signal outputted from the light-receiving element 100b, which makes it possible to acquire information about a corresponding part of the living body 1 with high accuracy.

In addition, a plurality of light-receiving elements 100b is included, and the extracting section 200a extracts, from signals outputted from the plurality of light-receiving elements 100b, information about a plurality of parts corresponding to the signals. This makes it possible to individually acquire information about the plurality of parts.

The light-emitting element 100a and the light-receiving elements 100b are disposed in the same housing 150. This makes it possible to provide a living body information measurement apparatus having high usability.

The plurality of parts described above includes a plurality of blood vessels different from each other. This makes it possible to acquire, for example, the state of autonomic nerves, which is one example of the state of the living body 1, with high accuracy.

The state of the living body 1 is the state of autonomic nerves of the living body 1 . This allows the living body information measurement apparatus to output a result of determination of the state of autonomic nerves.

The living body information measuring method using the living body information measurement apparatus includes a step of individually detecting light applied to the living body 1 and scattered by a plurality of parts in the living body 1, and a step of discriminating the state of the living body 1 on the basis of a detection result for each of the parts in the detecting step.

This makes it possible to discriminate the state of the living body 1 on the basis of a detection result for each of the parts of the living body 1 in the detecting step, which makes it possible to discriminate the state of the living body 1 with high accuracy.

### [Living Body Information Measurement Apparatus According to Example 2]

### <<Configuration of Living Body Information Measurement Apparatus According to Example 2>>

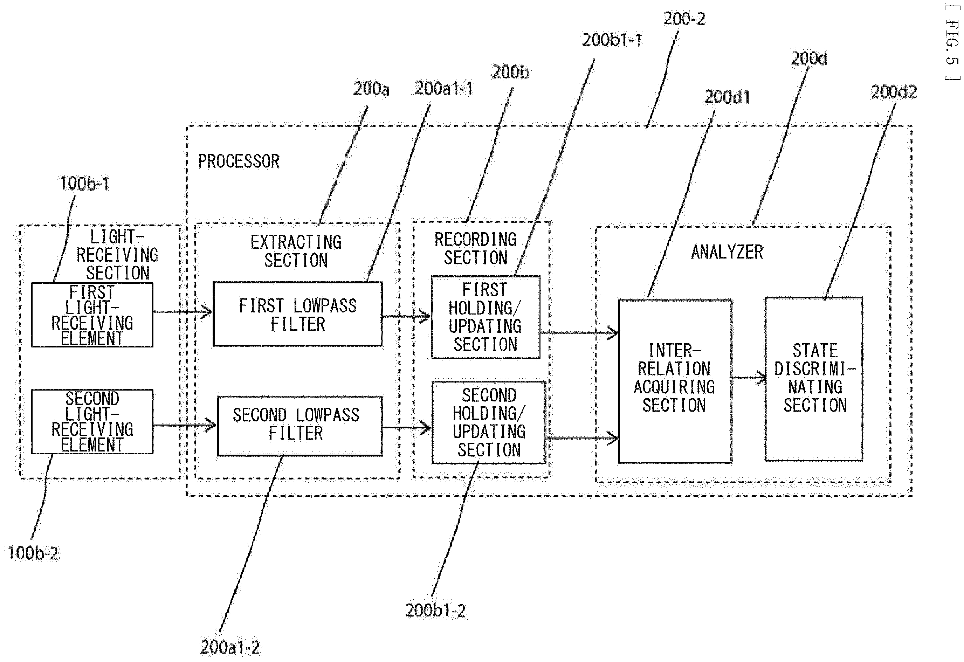

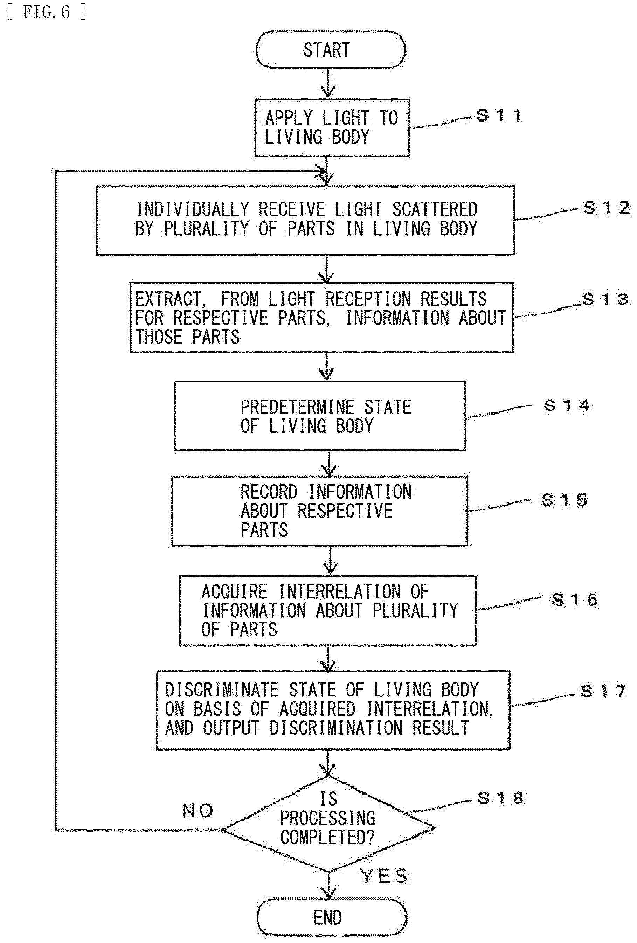

Description is given below of the living body information measurement apparatus according to Example 2 of the first embodiment of the present technology with reference to a block diagram in FIG. 5 and a flowchart in FIG. 6.

The living body information measurement apparatus has a configuration similar to that of the living body information measurement apparatus according to Example 1 described above, except that a configuration of an analyzer 200d of a processor 200-2 is different and a signal is not directly transmitted from the extracting section 200a to the analyzer 200d, as illustrated in FIG. 5.

In the living body information measurement apparatus according to Example 2, the analyzer 200d acquires a correlation (an interrelation) of information about a plurality of parts recorded on the recording section 200b, and discriminates the state of the living body 1 on the basis of a thus-acquired result.

The analyzer 200d includes an interrelation acquiring section 200d1 and a state discriminating section 200d2.

The interrelation acquiring section 200d1 continuously acquires a correlation between information about one part (e.g., the blood vessel 1a) in the living body 1 held or updated by the first holding/updating section 200b 1-1 and information about another part (e.g., the blood vessel 1b) in the living body held or updated by the second holding/updating section 200b 1-2, and transmits the correlation to the state discriminating section 200d2.

Here, it is presumable that the state (e.g., the state of autonomic nerves) of the living body 1 continues in the same state while the correlation between the information about the part described above and information about the other part described above is maintained in the same state. When the correlation between the information about the one part described above and the information about the other part described above has changed, it is presumable that the state (e.g., the state of autonomic nerves) of the living body 1 has changed.

Accordingly, the state discriminating section 200d2 determines that the state (e.g., the state of autonomic nerves) of the living body 1 is maintained in the same state while the correlation acquired by the interrelation acquiring section 200d1 is maintained in the same state, and outputs a result of discrimination (e.g., the rest state or the tension state) corresponding to a result of such determination.

### <<Operation of Living Body Information Measurement Apparatus According to Example 2>>

Description is given below of an operation of the living body information measurement apparatus according to Example 2 (an example of the living body information measuring method according to the present technology) with reference to a block diagram in FIG. 5 and a flowchart in FIG. 6.

It is assumed that the sensor device 100-1 of the living body information measurement apparatus according to Example 2 is also attached to the living body 1 as illustrated in FIG. 1B, for example.

In a first step S11, the light-emitting element 100a applies light to the living body 1. Specifically, when the living body information measurement apparatus is activated, the light-emitting element 100a emits light to apply the light to the living body 1.

In the next step S12, a plurality of light-receiving elements 100b individually receives light scattered by a plurality of parts (a plurality of blood vessels 1a and 1b) in the living body 1.

In the next step S13, the extracting section 200a extracts, from output signals of the light-receiving elements 100b, information about respective corresponding parts.

Specifically, an output signal of the first light-receiving element 100b-1 is filtered by the first lowpass filter 200a1-1 to cut a high-frequency component (a noise component), thereby extracting pulsation (a pulse wave) of the blood vessel 1a.

An output signal of the second light-receiving element 100b-2 is filtered by the second lowpass filter 200a1-2 to cut a high frequency, thereby extracting pulsation (a pulse wave) of the blood vessel 1b.

In the next step S14, the controller predetermines the state of the living body 1. Specifically, the controller calculates the heart rate of the living body on the basis of at least one of the signal having passed through the first lowpass filter 200a1-1 or the signal having passed through the second lowpass filter 200a1-2, and predetermines the state of autonomic nerves of the living body 1 (e.g., the rest state or the tension state) in accordance with a result of such calculation.

In the next step S15, the recording section 200b records the information about the respective parts extracted by the extracting section 200a in accordance with a result of determination in the step S4.

Specifically, in a case where a result of determination that the state of autonomic nerves of the living body 1 is, for example, the rest state (or the tension state) is acquired in the step S4, the first holding/updating section 200b1-1 and the second holding/updating section 200b2 associate the information about the respective parts (e.g., pulse waves of the blood vessels 1a and 1b) extracted by the extracting section 200a with the rest state (or the tension state), and hold or update the information.

In the next step S16, the analyzer 200d acquires a correlation (an interrelation) of information about a plurality of parts. Specifically, the interrelation acquiring section 200d1 acquires a correlation between information recorded (held or updated) on the first holding/updating section 200b1-1 and information recorded (held or updated) on the second holding/updating section 200b1-2.

In the next step S17, the state discriminating section 200d2 of the analyzer 200d discriminates the state (e.g., the state of autonomic nerves) of the living body 1 on the basis of the correlation acquired in the step S16, and outputs a result of such discrimination (e.g., the rest state or the tension state).

In the last step S18, whether or not processing is completed is determined. Determination here is affirmed when the operation of the living body information measurement apparatus is stopped, and is negated when the operation is maintained. In a case where determination in the step S18 is affirmed, the flow ends, and in a case where the determination in the step S18 is negated, the flow returns to the step S11.

### <<Effects of Living Body Information Measurement Apparatus and Living Body Information Measuring Method According to Example 2>>

According to the living body information measurement apparatus and the living body information measuring method according to Example 2 described above, the state of the living body 1 is discriminated on the basis of a correlation (an interrelation) of information about a plurality of parts, which makes it possible to discriminate the state of the living body 1 with low latency.

### [Living Body Information Measurement Apparatus According to Example 3]

### <<Configuration of Living Body Information Measurement Apparatus According to Example 3>>

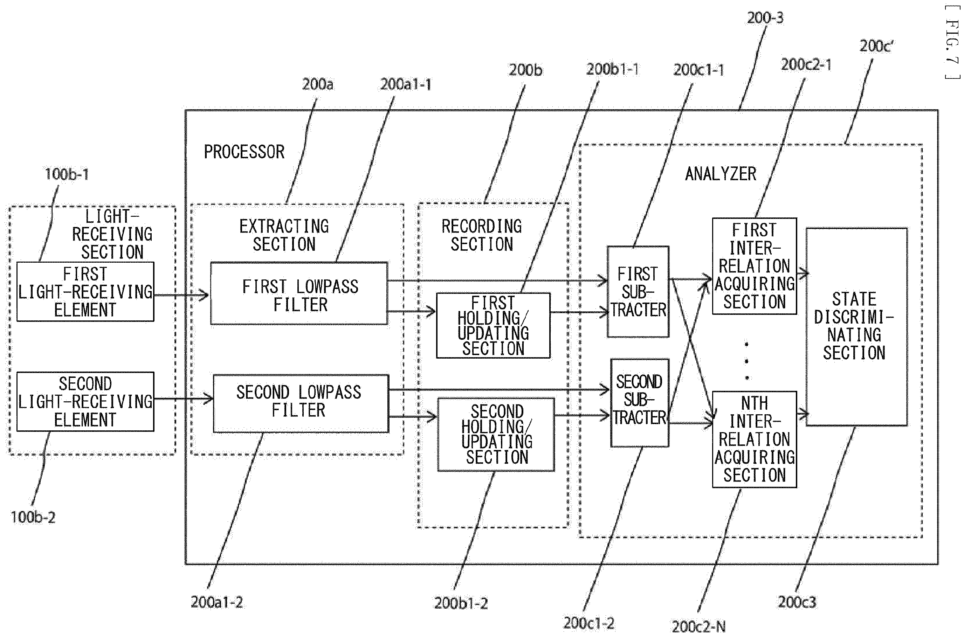

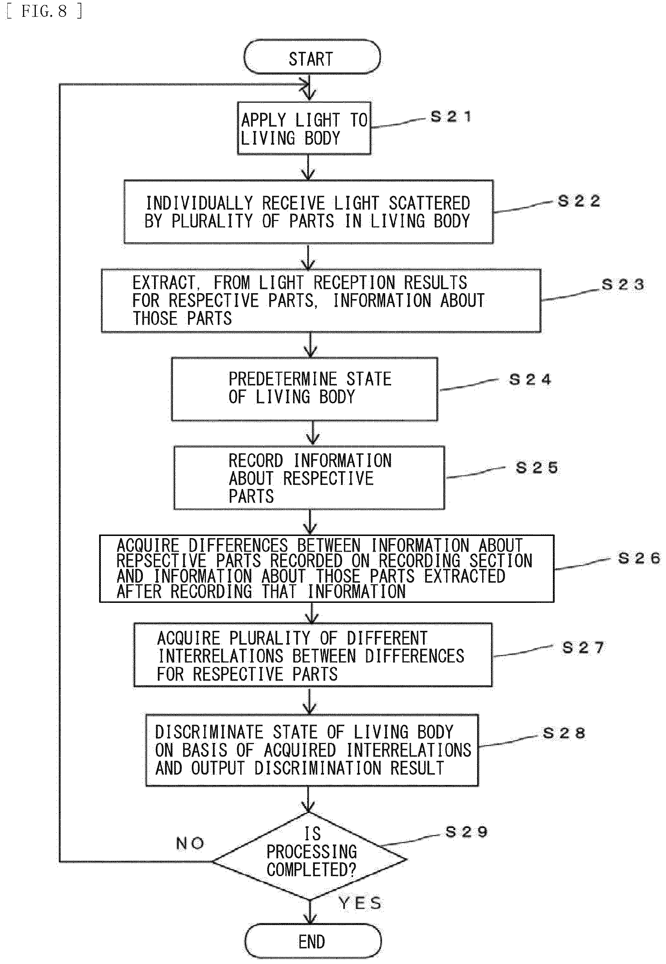

Description is given below of the living body information measurement apparatus according to Example 3 of the first embodiment of the present technology with a block diagram in FIG. 7 and a flowchart in FIG. 8.

The living body information measurement apparatus has a configuration similar to that of the living body information measurement apparatus according to Example 1 described above, except that a configuration of an analyzer 200c' of a processor 200-3 is different as illustrated in FIG. 7.

In the living body information measurement apparatus, the analyzer 200c' acquires a plurality of interrelations that are different in a result of subtraction (a difference) for each part, and discriminates the state of the living body 1 on the basis of a thus-acquired result.

To be more specific, the analyzer 200c' includes a first interrelation acquiring section 200c2-1 to an Nth interrelation acquiring section 200c2-N (N ≥ 2).

Respective interrelation acquiring sections 200c2-n (1 ≤ n ≤ N) acquire interrelations (the characteristic amounts described above) that are different from each other, and are interrelations between results of subtraction (differences) from the first and second subtracters 200c1-1 and 200c1-2.

### <<Operation of Living Body Information Measurement Apparatus According to Example 3>>

Description is given below of an operation of the living body information measurement apparatus according to Example 3 (an example of the living body information measuring method according to the present technology) with reference to a block diagram in FIG. 7 and a flowchart in FIG. 8.

It is assumed that the sensor device 100-1 of the living body information measurement apparatus according to Example 3 is also attached to the living body 1 as illustrated in FIG. 1B, for example.

In a first step S21, the light-emitting element 100a applies light to the living body 1. Specifically, when the living body information measurement apparatus is activated, the light-emitting element 100a emits light to apply the light to the living body 1.

In the next step S22, a plurality of light-receiving elements 100b individually receives light scattered by a plurality of parts (a plurality of blood vessels 1a and 1b) in the living body 1.

In the next step S23, the extracting section 200a extracts, from output signals of the light-receiving elements 100b, information about respective corresponding parts.

Specifically, an output signal of the first light-receiving element 100b-1 is filtered by the first lowpass filter 200a1-1 to cut a high-frequency component (a noise component), thereby extracting pulsation (a pulse wave) of the blood vessel 1a.

An output signal of the second light-receiving element 100b-2 is filtered by the second lowpass filter 200a1-2 to cut a high frequency, thereby extracting pulsation (a pulse wave) of the blood vessel 1b.

In the next step S24, the controller predetermines the state of the living body 1. Specifically, the controller calculates the heart rate of the living body on the basis of at least one of the signal having passed through the first lowpass filter 200a1-1 or the signal having passed through the second lowpass filter 200a1-2, and predetermines the state of autonomic nerves of the living body 1 (e.g., the rest state or the tension state) in accordance with a result of such calculation.

In the next step S25, the recording section 200b records the information about the respective parts extracted by the extracting section 200a in accordance with a result of determination in the step S24.

Specifically, in a case where a result of determination that the state of autonomic nerves of the living body 1 is, for example, the rest state (or the tension state) is acquired in the step S24, the first holding/updating section 200b1-1 and the second holding/updating section 200b2 associate the information about the respective parts (e.g., pulse waves of the blood vessels 1a and 1b) extracted by the extracting section 200a with the rest state (or the tension state), and hold or update the information.

In the next step S26, the analyzer 200c' subtracts the information about the respective parts recorded on the recording section 200b from information about those parts extracted by the extracting section 200a after recording that information. Specifically, the first subtracter 200c1-1 subtracts a signal recorded (held or updated) on the first holding/updating section 200b1-1 from a signal transmitted through the first lowpass filter 200a1-1 after a lapse of a predetermined time from when that signal is recorded. The second subtracter 200c1-2 subtracts a signal recorded (held or updated) on the second holding/updating section 200b1-2 from a signal transmitted through the second lowpass filter 200a1-2 after a lapse of a predetermined time from when that signal is recorded.

In the next step S27, the analyzer 200c' acquires a plurality of different interrelations between results of subtraction (differences) for the respective parts. Specifically, respective interrelation acquiring sections acquire interrelations (characteristic amounts described above) that are different from each other, and are interrelations (the characteristic amounts described above) between the result of subtraction in the first subtracter 200c1-1 and the result of subtraction in the second subtracter 200c1-2.

In the next step S28, the analyzer 200c' discriminates the state (e.g., the state of autonomic nerves) of the living body 1 on the basis of the plurality of interrelations that is different from each other and is acquired in the step S27, and outputs a result of such discrimination (e.g., the rest state or the tension state).

In the last step S29, whether or not processing is completed is determined. Determination here is affirmed when the operation of the living body information measurement apparatus is stopped, and is negated when the operation is maintained. In a case where determination in the step S29 is affirmed, the flow ends, and in a case where the determination in the step S29 is negated, the flow returns to the step S21.

### <<Effects of Living Body Information Measurement Apparatus and Living Body Information Measuring Method According to Example 3>>

According to the living body information measurement apparatus and the living body information measuring method according to Example 3 described above, the state of the living body 1 is discriminated on the basis of a plurality of different interrelations between results of subtraction for information about respective parts, which makes it possible to discriminate the state of the living body 1 with higher accuracy.

### [Living Body Information Measurement Apparatus According to Example 4]

### <<Configuration of Living Body Information Measurement Apparatus According to Example 4>>

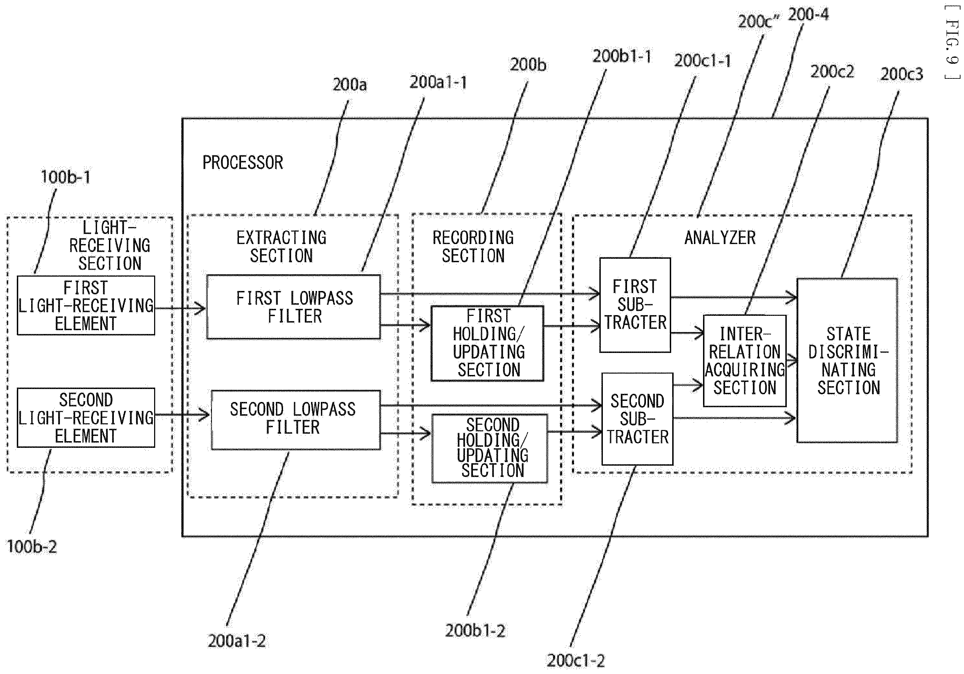

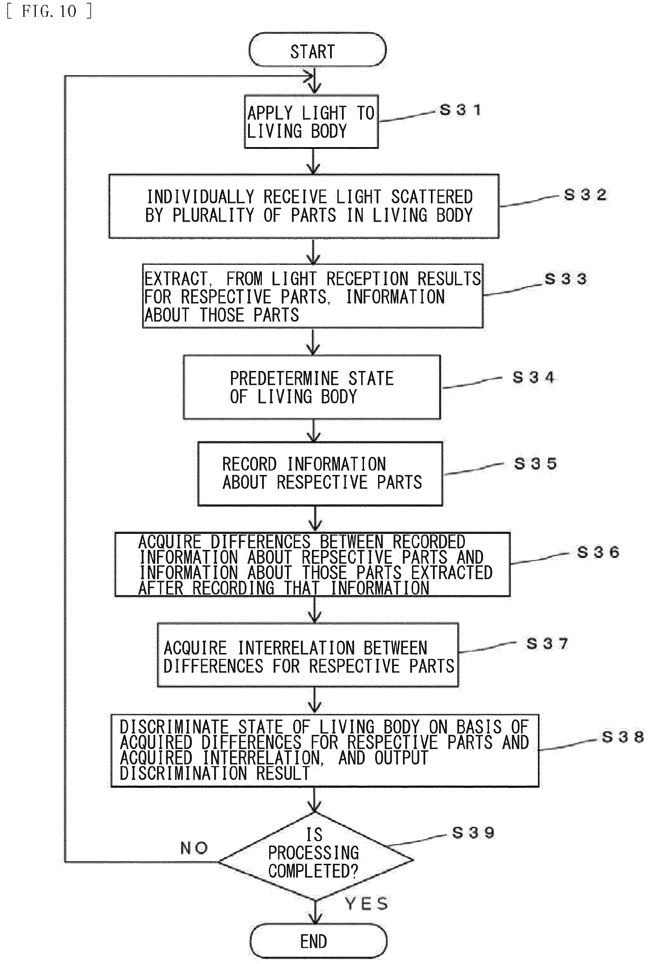

Description is given below of the living body information measurement apparatus according to Example 4 of the first embodiment of the present technology with reference to a block diagram in FIG. 9 and a flowchart in FIG. 10.

The living body information measurement apparatus according to Example 4 has a configuration similar to that of the living body information measurement apparatus 10-1 according to Example 1 described above, except that a configuration of an analyzer 200c" of a processor 200-4 is different as illustrated in FIG. 9.

In the living body information measurement apparatus according to Example 4, the analyzer 200c" acquires results of subtraction (differences) for information for respective parts and an interrelation between results of subtraction (differences) for information about a plurality of parts, and discriminates the state of the living body 1 on the basis of a thus- acquired result.

### <<Living Body Information Measurement Apparatus According to Example 4>>

Description is given below of an operation of the living body information measurement apparatus according to Example 4 (an example of the living body information measuring method according to the present technology) with reference to a block diagram in FIG. 9 and a flowchart in FIG. 10.

It is assumed that the sensor device 100-1 of the living body information measurement apparatus according to Example 4 is also attached to the living body 1 as illustrated in FIG. 1B, for example.

In a first step S31, the light-emitting element 100a applies light to the living body 1. Specifically, when the living body information measurement apparatus 10 is activated, the light-emitting element 100a emits light to apply the light to the living body 1.

In the next step S32, a plurality of light-receiving elements 100b individually receives light scattered by a plurality of parts (a plurality of blood vessels 1a and 1b) in the living body 1.

In the next step S33, the extracting section 200a extracts, from output signals of the light-receiving elements 100b, information about respective corresponding parts.

Specifically, an output signal of the first light-receiving element 100b-1 is filtered by the first lowpass filter 200a1-1 to cut a high-frequency component (a noise component), thereby extracting pulsation (a pulse wave) of the blood vessel 1a.

An output signal of the second light-receiving element 100b-2 is filtered by the second lowpass filter 200a1-2 to cut a high frequency, thereby extracting pulsation (a pulse wave) of the blood vessel 1b.

In the next step S34, the controller predetermines the state of the living body 1. Specifically, the controller calculates the heart rate of the living body on the basis of at least one of the signal having passed through the first lowpass filter 200a1-1 or the signal having passed through the second lowpass filter 200a1-2, and predetermines the state of autonomic nerves of the living body 1 (e.g., the rest state or the tension state) in accordance with a result of such calculation.

In the next step S35, the recording section 200b records the information about the respective parts extracted by the extracting section 200a in accordance with a result of determination in the step S34.

Specifically, in a case where a result of determination that the state of autonomic nerves of the living body 1 is, for example, the rest state (or the tension state) is acquired in the step S34, the first holding/updating section 200b1-1 and the second holding/updating section 200b2 associate the information about the respective parts (e.g., pulse waves of the blood vessels 1a and 1b) extracted by the extracting section 200a with the rest state (or the tension state), and hold or update the information.

In the next step S36, the analyzer 200c" acquires differences between information about respective parts recorded on the recording section 200b and information about those parts extracted by the extracting section 200a after recording that information. Specifically, the first subtracter 200c1-1 subtracts a signal recorded (held or updated) on the first holding/updating section 200b1-1 from a signal transmitted through the first lowpass filter 200a1-1 after a lapse of a predetermined time from when that signal is recorded. The second subtracter 200c1-2 subtracts a signal recorded (held or updated) on the second holding/updating section 200b1-2 from a signal transmitted through the second lowpass filter 200a1-2 after a lapse of a predetermined time from when that signal is recorded.

In the next step S37, the analyzer 200c' acquires an interrelation between results of subtraction (differences) for respective parts. Specifically, the interrelation acquiring section 200c2 acquires an interrelation between the result of subtraction in the first subtracter 200c1-1 and the result of subtraction in the second subtracter 200c1-2.

In the next step S38, the analyzer 200c" discriminates the state (e.g., the state of autonomic nerves) of the living body 1 on the basis of the differences for respective parts acquired in the step S36 and the interrelation acquired in the step S37, and outputs a result of such discrimination (e.g., the rest state or the tension state).

In the last step S29, whether or not processing is completed is determined. Determination here is affirmed when the operation of the living body information measurement apparatus is stopped, and is negated when the operation is maintained. In a case where determination in the step S39 is affirmed, the flow ends, and in a case where the determination in the step S39 is negated, the flow returns to the step S31.

### <<Effects of Living Body Information Measurement Apparatus and Living Body

### Information Measuring Method According to Example 4>>

According to the living body information measurement apparatus and the living body information measuring method according to Example 4 described above, the state of the living body is discriminated on the basis of differences in information for respective parts and a correction between the differences, which makes it possible to discriminate the state of the living body 1 with higher accuracy.

### [Living Body Information Measurement Apparatus According to Example 5]

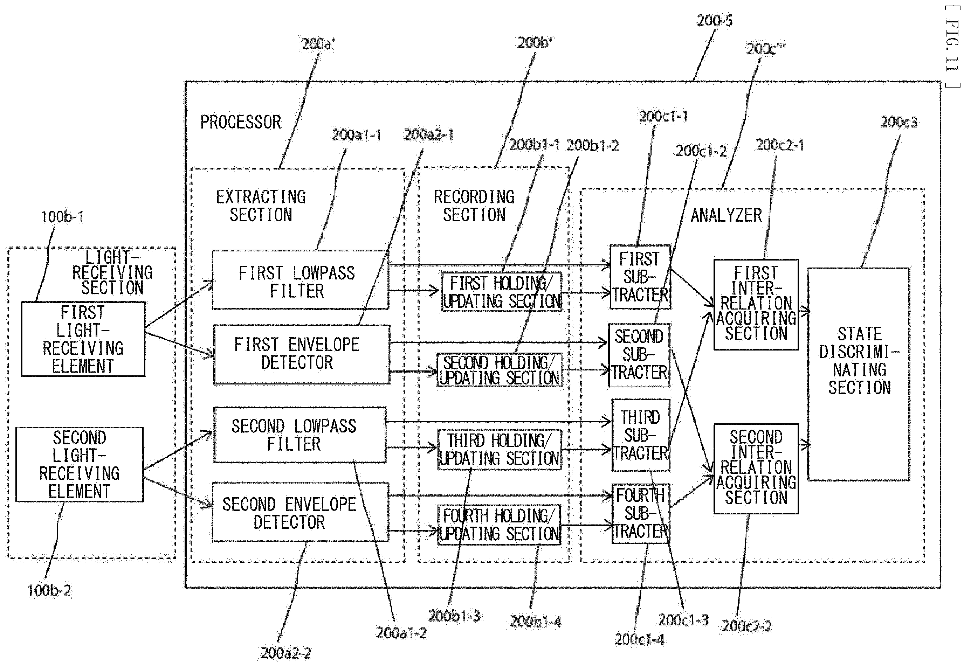

Description is given below of the living body information measurement apparatus according to Example 5 of the first embodiment of the present technology with reference to a block diagram in FIG. 11.

The living body information measurement apparatus according to Example 5 has a configuration similar to that of the living body information measurement apparatus according to Example 1 described above, except that configurations of an extracting section 200a' and an analyzer 200c'" of a processor 200-5 are different as illustrated in FIG. 11.

In the living body information measurement apparatus according to Example 5, the extracting section 200a' includes the first lowpass filter 200a1-1 and a first envelope detector 200a2-1 to which a signal from the first light-receiving element 100b-1 is to be transmitted, and the second lowpass filter 200a1-2 and a second envelope detector 200a2-2 to which a signal from the second light-receiving element 100b-2 is to be transmitted.

A signal outputted from the first light-receiving element 100b-1 and having passed through the first lowpass filter 200a1-1 is held or updated by the first holding/updating section 200b1-1. After a lapse of a predetermined time, the first subtracter 200c1-1 calculates a difference between the signal held or updated by the first holding/updating section 200b1-1 and a signal having passed through the first lowpass filter 200a1-1.

A signal outputted from the first light-receiving element 100b-1 and having passed through the first envelope detector 200a2-1 is held or updated by the second holding/updating section 200b1-2. After a lapse of a predetermined time, the second subtracter 200c1-2 calculates a difference between the signal held or updated by the second holding/updating section 200b1-1 and a signal having passed through the first envelope detector 200a2-1.

A signal outputted from the second light-receiving element 100b-2 and having passed through the second lowpass filter 200a1-2 is held or updated by a third holding/updating section 200b1-3. After a lapse of a predetermined time, a third subtracter 200c1-3 calculates a difference between the signal held or updated by the third holding/updating section 200b1-3 and a signal having passed through the second lowpass filter 200a1-2.

A signal outputted from the second light-receiving element 100b-2 and having passed through the second envelope detector 200a2-2 is held or updated by a fourth holding/updating section 200b1-4. After a lapse of a predetermined time, a fourth subtracter 200c1-4 calculates a difference between the signal held or updated by the fourth holding/updating section 200b1-4 and a signal having passed through the second envelope detector 200a2-2.

A result of subtraction in the first subtracter 200c1-1 and a result of subtraction in the third subtracter 200c1-3 are transmitted to first interrelation acquiring section 200c2-1.

The first interrelation acquiring section 200c2-1 acquires an interrelation between signals from which noise is cut for respective parts.

The result of subtraction in the second subtracter 200c1-2 and the result of subtraction in the fourth subtracter 200c1-4 are transmitted to the second interrelation acquiring section 200c2-2.

The second interrelation acquiring section 200c2-2 acquires an interrelation between envelopes of signals for respective parts.

The state discriminating section 200c3 discriminates the state (e.g., the state of autonomic nerves) of the living body 1 on the basis of the signals from which noise is cut for respective parts from the first interrelation acquiring section 200c2-1 and the envelopes of the signals for respective parts from the second interrelation acquiring section 200c2-2, and outputs a result of such discrimination (e.g., the rest state or the tension state).

According to the living body information measurement apparatus according to Example 5 described above, the state of the living body 1 is discriminated on the basis of an interrelation between the results of subtraction for signals from which noise is cut and an interrelation between results of subtraction for envelopes of signals, which makes it possible to discriminate the state of the living body 1 with higher accuracy.

### [Living Body Information Measurement Apparatus According to Example 6]

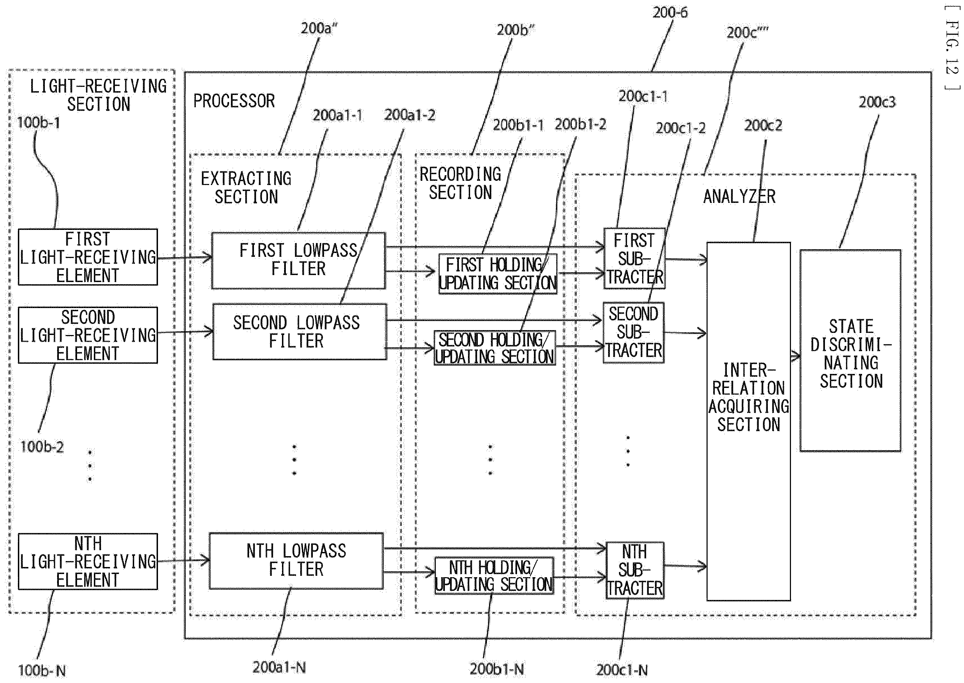

Description is given below of the living body information measurement apparatus according to Example 6 of the first embodiment of the present technology with reference to a block diagram in FIG. 12.

In the living body information measurement apparatus according to Example 6, as illustrated in FIG. 12, the light-receiving section includes N (N ≥ 3) light-receiving elements 100b (the first light-receiving element 100b-1 to an Nth light-receiving element 100b-N). An extracting section 200a" of a processor 200-6 includes N lowpass filters 200a1 (the first lowpass filter 200a1-1 to an Nth lowpass filter 200a1-N). A recording section 200b" of the processor 200-6 includes N holding/updating sections 200b1 (the first holding/updating section 200b1-1 to the second holding/updating section 200b1-N). An analyzer 200c"" of the processor 200-6 includes N subtracters 200c1 (the first subtracter 200c1-1 to an Nth subtracter 200c1-N).

That is, in the living body information measurement apparatus according to Example 6, the number of channels is larger, as compared with the living body information measurement apparatus according to Example 1, which makes it possible to acquire a highly accurate and highly reliable interrelation, and by extension to discriminate the state of the living body 1 with higher accuracy.

### 3. <Living Body Information Measurement Apparatus According to Second Embodiment of Present Technology>

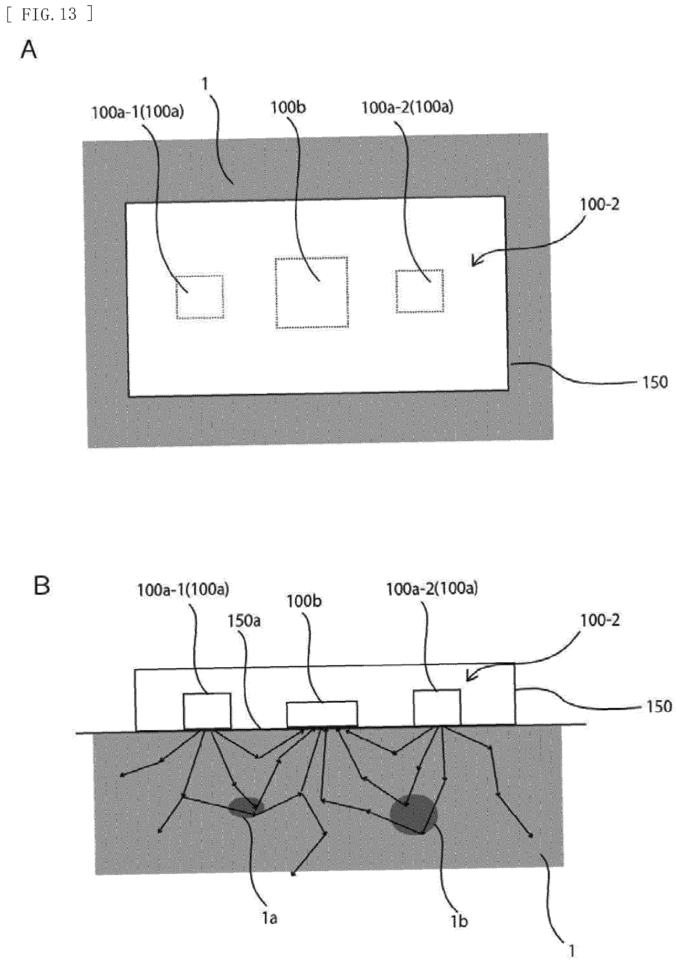

Description is given of a living body information measurement apparatus according to a second embodiment of the present technology with reference to FIG. 13A and FIG. 13B.

In the living body information measurement apparatus according to the second embodiment, as illustrated in FIG. 13A, a sensor device 100-2 has a configuration in which the numbers and arrangement of the light-emitting elements 100a and the light-receiving elements 100b are opposite to those in the living body information measurement apparatus according to the first embodiment.

To be more specific, the sensor device 100-2 includes a plurality of (e.g., two) light-emitting elements 100a (e.g., 100a-1 and 100a-2), and at least one (e.g., one) light-receiving element 100b.

The plurality of light-emitting elements 100a is disposed around the light-receiving element 100b, or more specifically at positions between which the light-receiving element 100b is sandwiched, on the light transmission window 150a in the housing 150.

For example, as illustrated in FIG. 13B, the sensor device 100-2 configured as described above is attached to the living body 1 to make a direction of arrangement of the plurality of (e.g., two) light-emitting elements 100a and the light-receiving element 100b substantially coincident with a direction of arrangement of two blood vessels 1a and 1b in the living body 1 and to position the light-receiving element 100b between the two blood vessels 1a and 1b as viewed from a direction facing the living body 1.

At this time, light emitted from the light-emitting elements 100a-1 and 100a-2 passes through the light transmission window 150a to be applied to the living body 1.

A portion of light that has been applied to the living body 1 from the light-emitting element 100a-1 and has entered the living body 1 propagates in the living body 1 to enter the blood vessel 1a, and is scattered by the blood vessel 1a. At least a portion of light scattered by the blood vessel 1a propagates in the living body 1 to enter the light-receiving element 100b.

A portion of light that has been applied to the living body 1 from the light-emitting element 100a-2 and has entered the living body 1 propagates in the living body 1 to enter the blood vessel 1b, and is scattered by the blood vessel 1b. At least a portion of the light scattered by the blood vessel 1b propagates in the living body 1 to enter the light-receiving element 100b.

Thus, the sensor device 100-2 is able to individually detect light scattered by each of the two blood vessels 1a and 1b in the living body 1.

That is, the sensor device 100-2 is able to separately detect the light scattered by the blood vessel 1a and the light scattered by the blood vessel 1b.

In this case, a signal outputted from the light-receiving element 100b that receives scattered light from the blood vessel 1a has a waveform corresponding to pulsation of the blood vessel 1a, and a signal outputted from the light-receiving element 100b that receives scattered light from the blood vessel 1b has a waveform corresponding to pulsation of the blood vessel 1b. Pulsation of a blood vessel is an index for determining the state (e.g., a state of autonomic nerves) of the living body 1.

It is to be noted that in the sensor device 100-2, the plurality of light-emitting elements 100a-1 and 100a-2 emit light at different timings, which makes it possible to suppress crosstalk (reception of light scattered by a blood vessel that is not a detection target).

### 4. <Living Body Information Measurement Apparatus According to Third Embodiment of Present Technology>

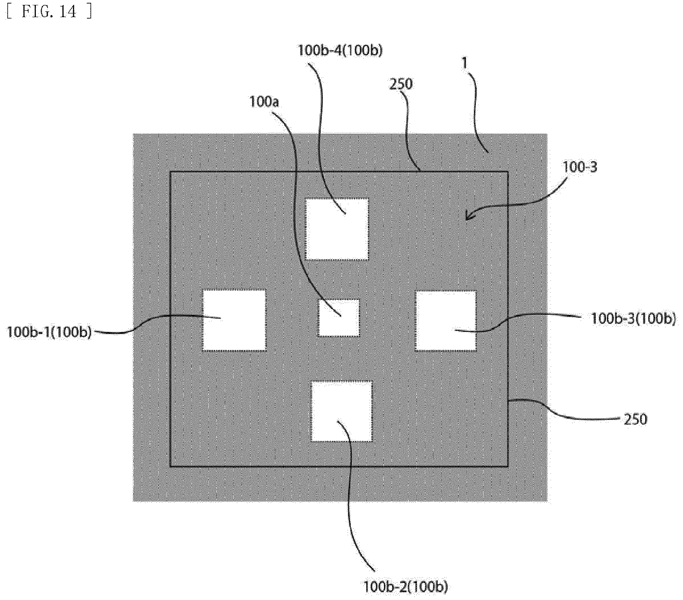

Description is given of a living body information measurement apparatus according to a third embodiment of the present technology (living body information measurement apparatuses according to Examples 1 to 3) with reference to FIGs. 14 to 16.

In the living body information measurement apparatus according to the third embodiment, as illustrated in FIGs. 14 to 16, the number of light-receiving elements 100b of each of sensor devices 100-3, 100-4, and 100-5, and arrangement of the light-emitting element 100a and the light-receiving elements 100b are different from those in the living body information measurement apparatuses according to the respective examples of the first embodiment described above.

To be more specific, in the sensor devices 100-3, 100-4, and 100-5, for example, as illustrated in FIGs. 14 o 16, a plurality of (e.g., three or more) light-receiving elements 100b is disposed around the light-emitting element 100a.

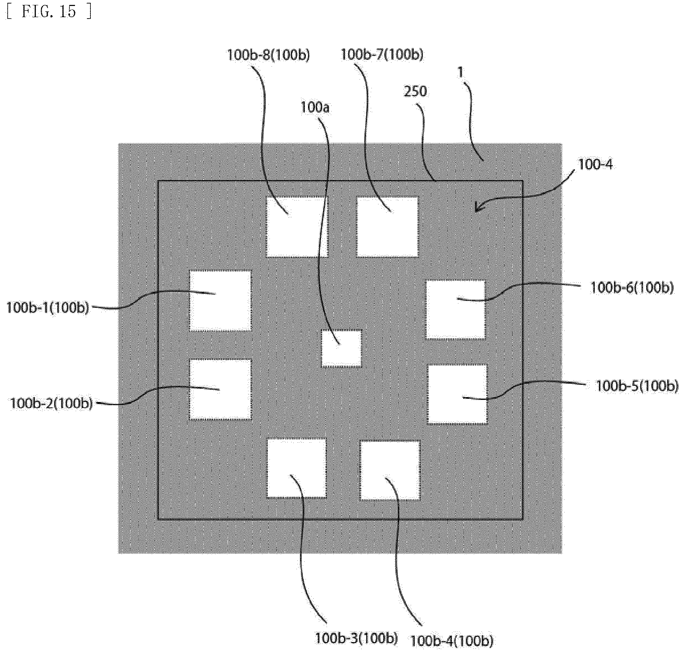

In the sensor devices 100-3 and 100-4 of the living body information measurement apparatuses according to Examples 1 and 2, for example, as illustrated in FIG. 14 and FIG. 15, a plurality of (e.g., three or more) light-receiving elements 100b is disposed to surround the light-emitting element 100a.

In the sensor device 100-3 of the living body information measurement apparatus according to Example 1, for example, as illustrated in FIG. 14, four light-receiving elements 100b (the first light-receiving element 100b-1 to a fourth light-receiving element 100b-4) are disposed to surround the light-emitting element 100a.

In the sensor device 100-4 of the living body information measurement apparatus according to Example 2, for example, as illustrated in FIG. 15, eight light-receiving elements 100b (the first light-receiving element 100b-1 to an eighth light-receiving element 100b-8) are disposed to surround the light-emitting element 100a.

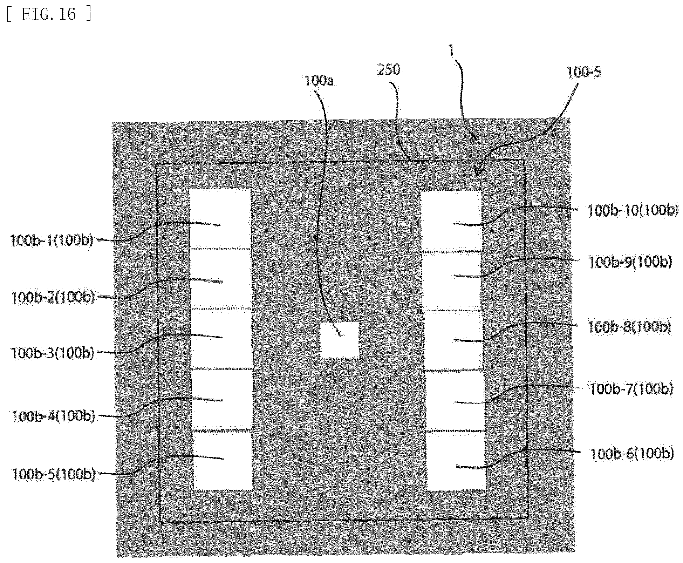

In the sensor device 100-5 of the living body information measurement apparatus according to Example 3, for example, as illustrated in FIG. 16, the light-emitting element 100a and a plurality of (e.g., ten) light-receiving elements 100b (the first light-receiving element 100b-1 to a tenth light-receiving element 100b-10) are disposed in the housing 250 to position the light-emitting element 100a between a light-receiving element group including a plurality of (e.g., five) light-receiving elements 100b arranged in one axis direction and a light-receiving element group including a plurality of (e.g., five) light-receiving elements 100b arranged in another axis direction parallel to the one axis direction.

According to the living body information measurement apparatuses according to the examples of the third embodiment described above, three or more light-receiving elements are able to individually receive light applied from the light-emitting element 100a to the living body 1 and scattered by three or more parts in the living body 1, which makes it possible to discriminate the state of the living body 1 with higher accuracy.

### 5. <Modification Examples of Present Technology>

The present technology is not limited to the configurations described in the above respective embodiments, and may be modified as appropriate.

For example, in the respective examples of the third embodiment described above, the light-emitting element and the light-receiving element may be replaced with each other.

In addition, in the respective embodiments described above, the state of the living body 1 that is a discrimination target of the living body information measurement apparatus is, for example, the state of autonomic nerves of the living body 1; however, instead of this, the state may be, for example, an endocrine state or an immune state of a living body.

In the respective embodiments described above, information about a plurality of parts recorded on the recording section 200b may be recorded in association with a predetermined state (e.g., the rest state or the tension state) of the living body 1 by performing calibration in advance with use of the living body information measurement apparatus.

In this case, it is not necessary to update information in the recording section 200b during operation.

In the respective embodiments described above, in a case where the controller predetermines the state of the living body 1, the controller acquires the heart rate of the living body 1; however, instead of this, predetermination may be performed, for example, by acquiring acceleration information (motion information about the living body) with use of an acceleration sensor, or acquiring perspiration information and the like with use of a humidity sensor.

In the respective embodiments described above, instead of the recording section, for example, a machine learning section may be provided that performs machine learning of information about a plurality of parts of the living body 1 extracted by the extracting section. In a case where the machine learning section is provided, in addition to the recording section, the analyzer may not be provided.

The machine learning section performs, for example, machine learning of information about respective parts of the living body 1 extracted in time series by the extracting section to estimate motions of the parts (e.g., pulsation of blood vessels), which makes it possible to discriminate the state of the living body 1 (e.g., the state of autonomic nerves) with high accuracy.

Performing prior learning in advance makes it possible for the machine learning section to immediately discriminate the state of the living body 1 (e.g., the state of autonomic nerves) with high accuracy from information about respective parts of the living body 1 extracted by the extracting section.

The machine learning section may perform, for example, such simple calculation as calculation of the characteristic amount described above from information about respective parts of the living body 1 extracted by the extracting section, and may transmit a result of such calculation to the analyzer. That is, the machine learning section may not necessarily perform discrimination of the state of the living body 1.

In addition, the present technology may have the following configurations.
(1) A living body information measurement apparatus including:
   a sensor device that applies light to a living body and individually detects light scattered by a plurality of parts in the living body; and
   a processor that discriminates a state of the living body on the basis of outputs of the sensor device for the respective parts.
(2) The living body information measurement apparatus according to (1), in which the sensor device includes at least one light-emitting element and a plurality of light-receiving elements, or a plurality of light-emitting elements and at least one light-receiving element.
(3) The living body information measurement apparatus according to (1) or (2), in which the processor includes an extracting section that extracts information about the plurality of parts from the outputs of the sensor device, and discriminates the state of the living body on the basis of the information about the plurality of parts extracted by the extracting section.
(4) The living body information measurement apparatus according to (3), in which the processor discriminates the state of the living body with use of information about the respective parts extracted in time series by the extracting section.
(5) The living body information measurement apparatus according to (3) or (4), in which the processor includes a recording section that records the information about the plurality of parts extracted by the extracting section.
(6) The living body information measurement apparatus according to any one of (3) to (5), in which the processor includes a machine learning section that performs machine learning of the information about the plurality of parts extracted by the extracting section.
(7) The living body information measurement apparatus according to any one of (3) to (6), in which the processor includes an analyzer that analyzes the information about the plurality of parts extracted by the extracting section and performs discrimination of the state of the living body on the basis of a result of such analysis.
(8) The living body information measurement apparatus according to (7), in which the analyzer acquires an interrelation of the information about the plurality of parts extracted by the extracting section, and performs the discrimination on the basis of a thus-acquired result.
(9) The living body information measurement apparatus according to (7), in which the analyzer calculates a difference between information about each of the parts extracted by the extracting section and information about that part extracted by the extracting section before extracting that information, and performs discrimination of the state of the living body on the basis of a result of such calculation.
(10) The living body information measurement apparatus according to (9), in which the analyzer acquires an interrelation between the differences in the information about the plurality of parts, and performs discrimination of the state of the living body on the basis of a thus-acquired result.
(11) The living body information measurement apparatus according to (10), in which the analyzer acquires a plurality of the interrelations, and performs discrimination of the state of the living body on the basis of a thus-acquired result.
(12) The living body information measurement apparatus according to (9), in which the analyzer acquires an interrelation between the differences in the information about the plurality of parts, and performs discrimination of the state of the living body on the basis of a thus-acquired result and the differences for the respective parts.
(13) The living body information measurement apparatus according to (5), in which
   the processor includes a determining section that determines whether or not the information about the plurality of parts extracted by the extracting section satisfies a condition indicating that the state of the living body is a predetermined state, and
   the recording section holds or updates the information for the respective parts when a result of determination in the determining section is affirmative.
(14) The living body information measurement apparatus according to (5), in which the information about the plurality of parts recorded on the recording section is recorded in association with a predetermined state of the living body by performing calibration in advance with use of the living body information measurement apparatus.
(15) The living body information measurement apparatus according to any one of (3) to (14), in which the extracting section includes a lowpass filter.
(16) The living body information measurement apparatus according to any one of (3) to (15), in which the extracting section includes an envelope detector.
(17) The living body information measurement apparatus according to any one of (2) to (16), in which
   a plurality of the light-emitting elements is included,
   the plurality of the light-emitting elements applies light to the living body at different timings, and
   the processor acquires, from a plurality of signals outputted by the light-receiving element at different timings, information about the plurality of parts corresponding the signals.
(18) The living body information measurement apparatus according to any one of (2) to (17), in which
   a plurality of the light-receiving elements is included, and
   the processor acquires, from signals outputted from the plurality of the light-receiving elements, information about the plurality of parts corresponding to the signals.
(19) The living body information measurement apparatus according to any one of (2) to (18), in which the light-emitting elements and the light-receiving elements are disposed in a same housing.
(20) The living body information measurement apparatus according to any one of (1) to (19), in which the plurality of parts includes a plurality of blood vessels different from each other.
(21) The living body information measurement apparatus according to any one of (1) to (20), in which the state of the living body includes a state of autonomic nerves of the living body.
(22) A living body information measuring method including:
   a step of applying light to a living body and individually detecting light scattered by a plurality of parts in the living body; and
   a step of discriminating a state of the living body on the basis of a detection result for each of the parts in the detecting step.

### Reference Signs List

1: living body
1a, 1b: blood vessel
100a: light-emitting element
100b: light-receiving element
100-1, 100-2, 100-3, 100-4, 100-5: sensor device
150, 250: housing
200-1, 200-2, 200-3, 200-4, 200-5, 200-6: processor
200a: extracting section
200a1: lowpass filter
200b: recording section
200b1: first holding/updating section
200c: analyzer
200c1: subtracter
200c2, 200d1: interrelation acquiring section
200c3, 200d2: state discriminating section

## Claims

1. A living body information measurement apparatus comprising:
a sensor device that applies light to a living body and individually detects light scattered by a plurality of parts in the living body; and
a processor that discriminates a state of the living body on a basis of outputs of the sensor device for the respective parts.

2. The living body information measurement apparatus according to claim 1, wherein the sensor device includes at least one light-emitting element and a plurality of light-receiving elements, or a plurality of light-emitting elements and at least one light-receiving element.

3. The living body information measurement apparatus according to claim 1, wherein the processor includes an extracting section that extracts information about the plurality of parts from the outputs of the sensor device, and discriminates the state of the living body on a basis of the information about the plurality of parts extracted by the extracting section.

4. The living body information measurement apparatus according to claim 3, wherein the processor discriminates the state of the living body with use of information about the respective parts extracted in time series by the extracting section.

5. The living body information measurement apparatus according to claim 3, wherein the processor includes a recording section that records the information about the plurality of parts extracted by the extracting section.

6. The living body information measurement apparatus according to claim 3, wherein the processor includes a machine learning section that performs machine learning of the information about the plurality of parts extracted by the extracting section.

7. The living body information measurement apparatus according to claim 3, wherein the processor includes an analyzer that analyzes the information about the plurality of parts extracted by the extracting section and performs discrimination of the state of the living body on a basis of a result of such analysis.

8. The living body information measurement apparatus according to claim 7, wherein the analyzer acquires an interrelation of the information about the plurality of parts extracted by the extracting section, and performs the discrimination on a basis of a thus-acquired result.

9. The living body information measurement apparatus according to claim 7, wherein the analyzer calculates a difference between information about each of the parts extracted by the extracting section and information about that part extracted by the extracting section before extracting that information, and performs discrimination of the state of the living body on a basis of a result of such calculation.

10. The living body information measurement apparatus according to claim 9, wherein the analyzer acquires an interrelation between the differences in the information about the plurality of parts, and performs discrimination of the state of the living body on a basis of a thus-acquired result.

11. The living body information measurement apparatus according to claim 10, wherein the analyzer acquires a plurality of the interrelations, and performs discrimination of the state of the living body on a basis of a thus-acquired result.

12. The living body information measurement apparatus according to claim 9, wherein the analyzer acquires an interrelation between the differences in the information about the plurality of parts, and performs discrimination of the state of the living body on a basis of a thus-acquired result and the differences for the respective parts.

13. The living body information measurement apparatus according to claim 5, wherein
the processor includes a determining section that determines whether or not the information about the plurality of parts extracted by the extracting section satisfies a condition indicating that the state of the living body is a predetermined state, and
the recording section holds or updates the information for the respective parts when a result of determination in the determining section is affirmative.

14. The living body information measurement apparatus according to claim 5, wherein the information about the plurality of parts recorded on the recording section is recorded in association with a predetermined state of the living body by performing calibration in advance with use of the living body information measurement apparatus.

15. The living body information measurement apparatus according to claim 3, wherein the extracting section includes a lowpass filter.

16. The living body information measurement apparatus according to claim 3, wherein the extracting section includes an envelope detector.

17. The living body information measurement apparatus according to claim 2, wherein
a plurality of the light-emitting elements is included,
the plurality of the light-emitting elements applies light to the living body at different timings, and
the processor acquires, from a plurality of signals outputted by the light-receiving element at different timings, information about the plurality of parts corresponding the signals.

18. The living body information measurement apparatus according to claim 2, wherein
a plurality of the light-receiving elements is included, and
the processor acquires, from signals outputted from the plurality of the light-receiving elements, information about the plurality of parts corresponding to the signals.

19. The living body information measurement apparatus according to claim 2, wherein the light-emitting elements and the light-receiving elements are disposed in a same housing.

20. The living body information measurement apparatus according to claim 1, wherein the plurality of parts comprises a plurality of blood vessels different from each other.

21. The living body information measurement apparatus according to claim 1, wherein the state of the living body comprises a state of autonomic nerves of the living body.

22. A living body information measuring method comprising:
a step of applying light to a living body and individually detecting light scattered by a plurality of parts in the living body; and
a step of discriminating a state of the living body on a basis of a detection result for each of the parts in the detecting step.
